# EUROPEAN PATENT APPLICATION

(11) **EP 4 748 326 A2**
(43) Date of publication of application: **27.05.2026**
(21) Application number: 26172181.5
(22) Date of filing: 22.12.2022
(51) Int. Cl.: A61B 17/42

(54) **ADJUSTMENT DEVICES FOR POSITIONING A UTERINE MANIPULATOR**

(30) Priority: 03.01.2022 US 202263295981 P
(62) Divisional of application: 22854291.6
(71) Applicant: CooperSurgical, Inc., Trumbull, CT 06611 (US)
(72) Inventor: IVOSEVIC, Milan, Kinnelon, New Jersey 07405 (US); PARADISE, Charles, Brooklyn, New York 11217 (US); SOTO, Orlando, Amesbury, Massachusetts 01913 (US); RODRIGUEZ, Christopher, Carlsbad, California 92009 (US); FALKENBURG, Cameron, San Diego, California 92117 (US); CROSS, Laura, Columbia, Maryland 21045 (US); MURPHY, Brian, Baltimore, Maryland 21218 (US); CHAUHAN, Raghuraj, Elkridge, Maryland 21075 (US)
(74) Representative: Fish & Richardson P.C.

(57) **Abstract**

An adjustment device for positioning a uterine manipulator includes a support component that is movable along an arcuate path defined by the adjustment device and an adjustment unit. The adjustment unit includes a base component that is secured to the support component and a rotational element that is secured to the base component and to the uterine manipulator such that the rotational element and the uterine manipulator secured thereto are movable along the arcuate path and rotatable with respect to the support component. A positioning system includes the adjustment device and a positioning arm that supports the adjustment device for positioning a uterine manipulator.

## Description

### CROSS REFERENCE TO RELATED APPLICATION

This application claims priority to U.S. Provisional Patent Application No. 63/295,981, filed on January 03, 2022, pursuant to 35 USC §119.

### TECHNICAL FIELD

This disclosure relates to adjustment devices for positioning a uterine manipulator during a surgical procedure.

### BACKGROUND

Uterine manipulators are medical instruments that are used for manipulating a patient's uterus during surgical procedures, such as a laparoscopic gynecologic surgery (e.g., a total laparoscopic hysterectomy (TLH) surgery). With a distal portion of a uterine manipulator advanced through the vaginal canal and into the uterus, the uterus can be manipulated through surgeon-controlled movements of a proximal portion of the uterine manipulator. The uterine manipulator may be securely positioned and held in place during the procedure by a uterine positioning system that is mounted to an operating table. During such a procedure, a surgeon often needs to slightly adjust the position of the uterus via the uterine manipulator, requiring the surgeon either to leave his or her workstation to reposition the manipulator or to ask an assistant to do the same. In either case, the procedure is interrupted. Furthermore, if the surgeon asks an assistant to perform the adjustment, the situation is further complicated because the adjustment is blind and based only on verbal instructions from the surgeon.

### SUMMARY

In general, this disclosure relates to adjustment devices for positioning a uterine manipulator during a surgical procedure.

In one aspect, an adjustment device for positioning a uterine manipulator includes An adjustment device for positioning a uterine manipulator includes a support component that is movable along an arcuate path defined by the adjustment device and an adjustment unit. The adjustment unit includes a base component that is secured to the support component and a rotational element that is secured to the base component and to the uterine manipulator such that the rotational element and the uterine manipulator secured thereto are movable along the arcuate path and rotatable with respect to the support component.

Embodiments may include one or more of the following features.

In some embodiments, the adjustment device is configured to be fixedly attached to a positioning arm of a uterine positioning system.

In some embodiments, the adjustment unit is configured to move forward and backward along the arcuate path with respect to the positioning arm.

In some embodiments, the adjustment device further includes internal electronics that are configured to effect wireless or wired communication with a remote controller.

In some embodiments, the adjustment device further includes a remote control unit that is configured to power and control one or more movement mechanisms of the adjustment device.

In some embodiments, the adjustment device further includes a support unit that provides an elongate receptacle, the elongate receptacle receiving the support component and defining the arcuate path.

In some embodiments, the support component includes a connector having an end that is retained within the elongate receptacle as the adjustment unit moves arcuately along the elongate receptacle.

In some embodiments, the adjustment unit is movable forward and backward along the arcuate path by a total distance of up to about 6 cm to up to about 26 cm.

In some embodiments, the rotational element includes a dial that is rotatable about a pin, and an angular span of the dial corresponds to a lateral range of a tip of the uterine manipulator of about 6 cm to about 36 cm.

In some embodiments, the support component defines the arcuate path.

In some embodiments, the adjustment device further includes one or more motors that control arcuate movement of the support component and rotation of the rotational element.

In some embodiments, the adjustment device further includes a translation assembly that is coupled to and causes linear movement of the support component.

In some embodiments, the adjustment device is configured to maintain rotation of a shaft of the uterine manipulator around a reference point as the support component moves along the arcuate path.

In some embodiments, the reference point is located at a patient's cervix.

In some embodiments, the adjustment device is configured such that rotation of the uterine manipulator by the rotational element is maintained about a reference point.

In some embodiments, the reference point is located at a patient's cervix, and wherein an axis of the adjustment unit intersects the reference point.

In another aspect, a positioning system includes a positioning arm and an adjustment device supported on the positioning arm for positioning a uterine manipulator. The adjustment device includes a support component that is movable along an arcuate path defined by the adjustment device and an adjustment unit. The adjustment unit includes a base component that is secured to the support component and a rotational element that is secured to the base component and to the uterine manipulator such that the rotational element and the uterine manipulator secured thereto are movable along the arcuate path and rotatable with respect to the support component.

Embodiments may include one or more of the following features.

In some embodiments, the positioning arm defines a global position of the adjustment device and wherein the adjustment device defines a local position of the uterine manipulator.

In some embodiments, the positioning system further includes a remote controller and a control box that communicates with the remote controller.

In another aspect, an adjustment device for positioning a uterine manipulator includes a rotatable support component that is secured to the uterine manipulator and rotatable about a rotational axis defined by the adjustment device. The adjustment device further includes a rotatable adjustment unit that is secured to an articulation control element of the uterine manipulator and to the rotatable support component such that the adjustment device is configured to rotate the uterine manipulator with respect to the rotational axis and to articulate a portion of the uterine manipulator with respect to the rotatable support component.

Embodiments may include one or more of the following features.

In some embodiments, the rotatable support component is coupled to a linear translation assembly.

In some embodiments, the linear translation assembly includes a rail and a carriage.

In some embodiments, the linear translation assembly is configured to be fixedly attached to a positioning arm of a uterine positioning system.

In some embodiments, the adjustment device further includes one or more mechanical actuation cables that are configured to selectively cause the rotatable support component to rotate in opposite rotational directions.

In some embodiments, the adjustment device further includes a remote motor that is coupled to and controls the one or more mechanical actuation cables.

In some embodiments, the adjustment device further includes one or more mechanical actuation cables that are configured to selectively cause the rotatable adjustment unit to rotate in opposite rotational directions.

In some embodiments, the adjustment device further includes a remote motor that is coupled to and controls the one or more mechanical actuation cables.

In some embodiments, the articulation control element includes a handle and wherein the portion includes a distal tip.

In some embodiments, the rotational axis intersects a patient's cervix.

In another aspect, a positioning system includes a positioning arm and an adjustment device supported on the positioning arm for positioning a uterine manipulator. The adjustment device includes a rotatable support component that is secured to the uterine manipulator and rotatable about a rotational axis defined by the adjustment device. The adjustment device further includes a rotatable adjustment unit that is secured to an articulation control element of the uterine manipulator and to the rotatable support component such that the adjustment device is configured to rotate the uterine manipulator with respect to the rotational axis and to articulate a portion of the uterine manipulator with respect to the rotatable support component.

In one aspect, an adjustment device for positioning a uterine manipulator includes a base, an adjustment unit, and an adapter. The base includes an elongate receptacle, and the adjustment unit is movable arcuately along the elongate receptacle. The adapter is secured to a rotational element of the adjustment unit and to the uterine manipulator such that arcuate movement of the adjustment unit and rotational movement of the rotational element respectively effect arcuate movement and rotational movement of the uterine manipulator.

Embodiments may include one or more of the following features.

In some embodiments, the base is fixedly attachable to a positioning arm of a positioning system.

In some embodiments, the elongate receptacle has a curved profile.

In some embodiments, the arcuate movement of the adjustment unit includes a horizontal component.

In some embodiments, the adjustment unit is configured to move forward and backward along the elongate receptacle.

In some embodiments, the adjustment unit is movable forward and backward by a total distance of up to about 6 cm to up to about 26 cm.

In some embodiments, the adjustment unit includes a connector having an end that is retained within the elongate receptacle of the base as the adjustment unit moves arcuately along the elongate receptacle.

In some embodiments, the rotational element includes a dial that is rotatable about an interior pin.

In some embodiments, the dial is rotatable by an angle of up to about 90 degrees to up to about 270 degrees.

In some embodiments, an angular span of the dial corresponds to a lateral range of a tip of the uterine manipulator of about 6 cm to about 36 cm.

In some embodiments, the adjustment device further includes internal electronics housed within one or both of the base and the adjustment unit.

In some embodiments, the internal electronics facilitate wireless communication with a remote controller.

In some embodiments, the internal electronics facilitate wired communication with a remote controller.

In another aspect, a positioning system includes a positioning arm and an adjustment device supported on the positioning arm for positioning a uterine manipulator. The adjustment device includes a base, an adjustment unit, and an adapter. The base includes an elongate receptacle, and the adjustment unit is movable arcuately along the elongate receptacle. The adapter is secured to a rotational element of the adjustment unit and to the uterine manipulator such that arcuate movement of the adjustment unit and rotational movement of the rotational element respectively effect arcuate movement and rotational movement of the uterine manipulator.

Embodiments may include one or more of the following features.

In some embodiments, the positioning arm defines a global position of the adjustment device.

In some embodiments, the positioning system further includes a remote controller.

In some embodiments, the adjustment device further included internal electronics that effect wireless communication with the remote controller.

In some embodiments, the internal electronics and a signal cable together effect wired communication with the remote controller.

In some embodiments, the adjustment unit is configured to move forward and backward along the elongate receptacle.

In some embodiments, the rotational element includes a dial that is rotatable about an interior pin.

Embodiments may provide one or more of the following advantages.

In some embodiments, remotely controlling robotic-like movements of the adjustment device can allow a surgeon to position a patient's uterus as desired for optimal cutting and removal access to the uterus without leaving his or her robotic surgery workstation at which a remote controller is operated. Thus, the surgeon can continue visualizing the patient's pelvic region on a display screen at the workstation while the adjustment device is remotely manipulated and without disruption. Such remote control of the adjustment device therefore advantageously results in a streamlined procedure with an improved effectiveness, an improved precision, and a reduced procedural time period.

Other aspects, features, and advantages will be apparent from the description, the drawings, and the claims.

### DESCRIPTION OF DRAWINGS

FIG. 1 is a perspective view of a positioning system that supports a uterine manipulator.
FIG. 2 is a perspective view of an adjustment device of the positioning system of FIG. 1.
FIG. 3 is a side view of the adjustment device of FIG. 2.
FIG. 4 is a side view of the adjustment device of FIG. 2 with an adjustment unit illustrated in three different arcuate positions.
FIG. 5 is a perspective view of the adjustment device of FIG. 2 illustrated with a rotational element in three different angular positions.
FIG. 6 is a perspective view of an alternative uterine manipulator that can be supported by the positioning system of FIG. 1.
FIG. 7 is a side view of an adjustment device in a reference linear configuration.
FIG. 8 is a side view of the adjustment device of FIG. 7 in an extended linear configuration.
FIG. 9 is a side view of the adjustment device of FIG. 7 in a reference arcuate configuration.
FIG. 10 is a side view of the adjustment device of FIG. 7 in an adjusted arcuate configuration.
FIG. 11 is a perspective view of the adjustment device of FIG. 7 in a first angular configuration.
FIG. 12 is a side view of the adjustment device of FIG. 7 in a second, opposite angular configuration.
FIG. 13 is a side view of an adjustment device in a reference linear configuration.
FIG. 14 is a side view of the adjustment device of FIG. 13 in an extended linear configuration.
FIG. 15 is a side view of the adjustment device of FIG. 13 in a first rotational configuration.
FIG. 16 is a side view of the adjustment device of FIG. 13 in a second rotational configuration that is opposite to the first rotational configuration.
FIG. 17 is a side view of the adjustment device of FIG. 13 in a third rotational configuration.
FIG. 18 is a side view of the adjustment device of FIG. 13 in a fourth rotational configuration that is opposite to the fourth rotational configuration.

### DETAILED DESCRIPTION

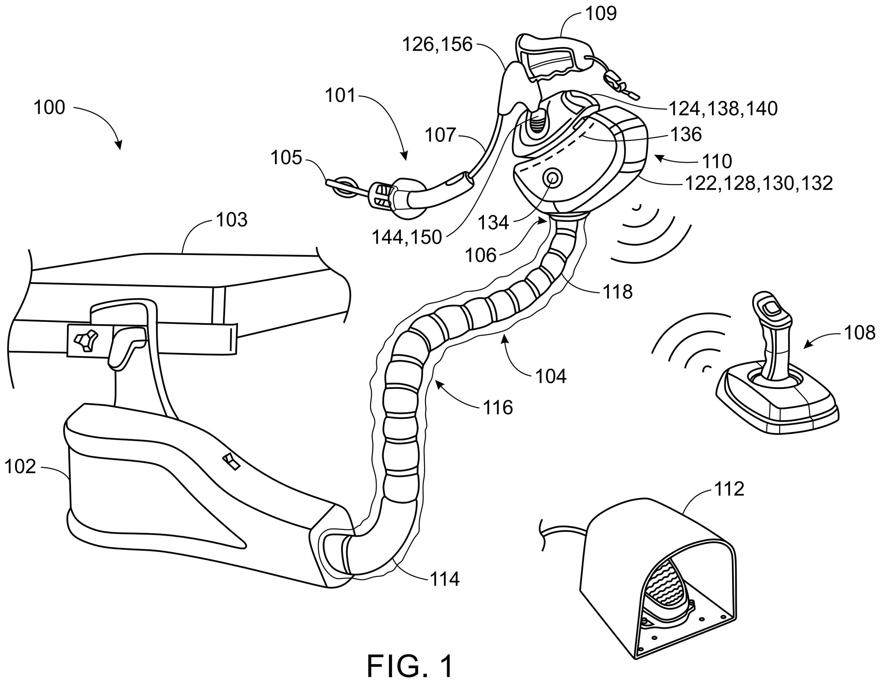

FIG. 1 illustrates a positioning system 100 that is designed to position a surgical tool for carrying out a surgical procedure. In some examples, the surgical procedure is a laparoscopic or robotic pelvic surgery for carrying out a total laparoscopic hysterectomy (TLH), and the surgical tool is a uterine manipulator 101 used to perform the TLH. The positioning system 100 is designed to be mounted to an operating table 103 and accordingly includes a mounting/powering unit 102. The positioning system 100 further includes a positioning arm 104 that extends from the mounting/powering unit 102, an adjustment device 110 that is attached to an upper end 106 of the positioning arm 104, a remote controller 108 for controlling certain components of the adjustment device 110, and a foot pedal 112 for locking and unlocking a configuration of the positioning arm 104.

The positioning arm 104 stably supports and securely positions the uterine manipulator 101 in place during the surgical procedure. The positioning arm 104 is embodied as a "gooseneck" structure that includes a rigid elbow section 114 and an adjustable section 116 to which the adjustment device 110 is attached. The adjustable section 116 includes multiple collars 118 (e.g., metal links) that are movable with respect to each other to provide the adjustable section 116 with a full range of motion. The positioning arm 104 also includes an internal tension cable (e.g., a braided mechanical cable) that extends from the upper end 106 of the positioning arm to the mounting/powering unit 102.

The foot pedal 112 can be depressed to unlock the positioning arm 104. That is, depression of the foot pedal 112 releases tension from the internal tension cable (e.g., deactivates the internal tension cable) to allow manual manipulation of the adjustable section 116 when desired. Conversely, the foot pedal 112 can be released from a depressed position to tension the internal tension cable (e.g., to activate the internal tension cable) for locking the configuration of the adjustable section 116. In the locked configuration, the collars 118 are positionally fixed, and the positioning arm 104 is rigid and immovable during the surgical procedure. Thus, in the locked configuration, the positioning arm 104 establishes a global position of the adjustment device 110, which is secured to its upper end 106. For locking and unlocking the positioning arm 104, the mounting/powering unit 102 includes a servo motor that is operable to pull and release the internal tension cable within the positioning arm 104. The mounting/powering unit 102 is powered via a wired connection to a wall plug.

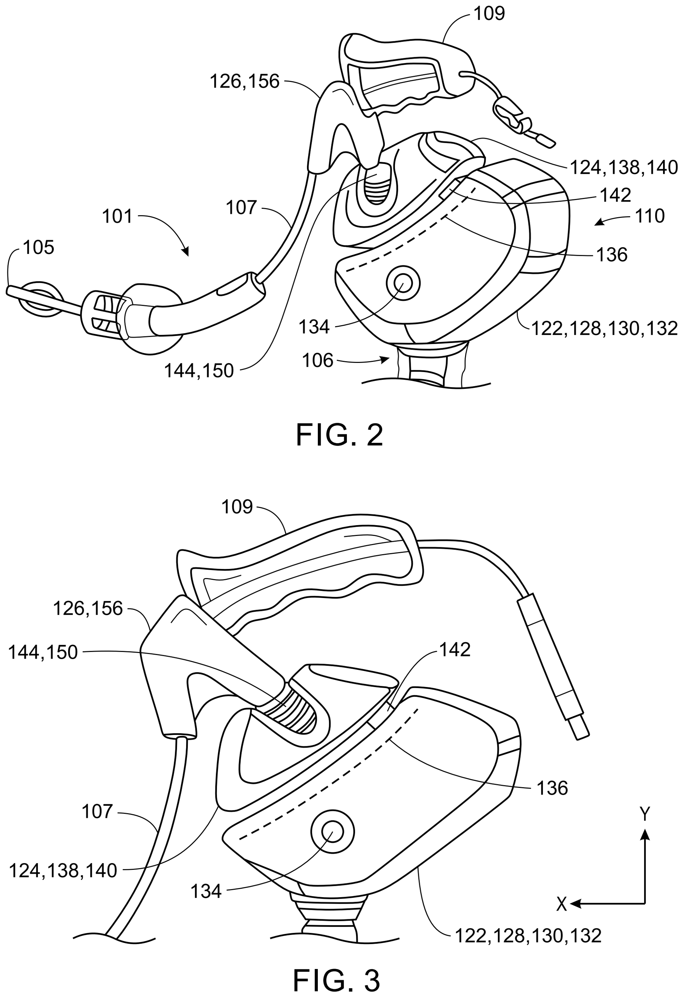

Referring to FIGS. 2 and 3, the adjustment device 110 is a robotic head that can further position (e.g., locally position) the uterine manipulator 101 with respect to the global position of the adjustment device 110. For example, once the positioning arm 104 has been locked in a desired configuration to establish a global position of the adjustment device 110, the adjustment device 110 can be operated with the remote controller 108 to fine-tune a position of the uterine manipulator 101, which is supported on the adjustment device 110. The adjustment device 110 includes a lower base 122 (e.g., a support unit) that is fixedly attached to the upper end 106 of the positioning arm 104, an upper adjustment unit 124 that is movable with respect to the base 122, and an adapter 126 that securely couples the adjustment unit 124 to the uterine manipulator 101.

The base 122 includes a housing 128, internal electronics 130, a rechargeable battery 132 that may be enclosed within the housing 128 for powering the adjustment device 110, and a power indicator light. Additionally, the base 122 may be connected directly to the remote controller 108 with a signal cable. The internal electronics 130 can effect wireless communication with the remote controller 108 and/or with the adjustment unit 124, as well as wired communication with the remote controller 108. The housing 128 carries a power selector 134 (e.g., a button or a switch) for powering the adjustment device 110 on and off. The base 122 further includes an elongate receptacle 136 (e.g., a rail or a slot indicated schematically with a dashed line that corresponds to an arcuate path) with a curved (e.g., arcuate) profile such that the adjustment unit 124 can undergo arcuate motion (e.g., curvilinear motion) in an *xy* plane along the elongate receptacle 136.

The adjustment unit 124 includes a housing 138 (e.g., a base component), internal electronics 140, a connector 142 (e.g., a support component) that moves along the elongate receptacle of the base 122, and a rotational element 144 (e.g., a pivotable element) that effectively allows the adapter 126 and the attached uterine manipulator 101 to be tilted from side to side. The internal electronics 140 can effect wireless communication with the remote controller 108 and/or with the base 122. In some embodiments, the internal electronics 140 can also effect wired communication with the remote controller 108.

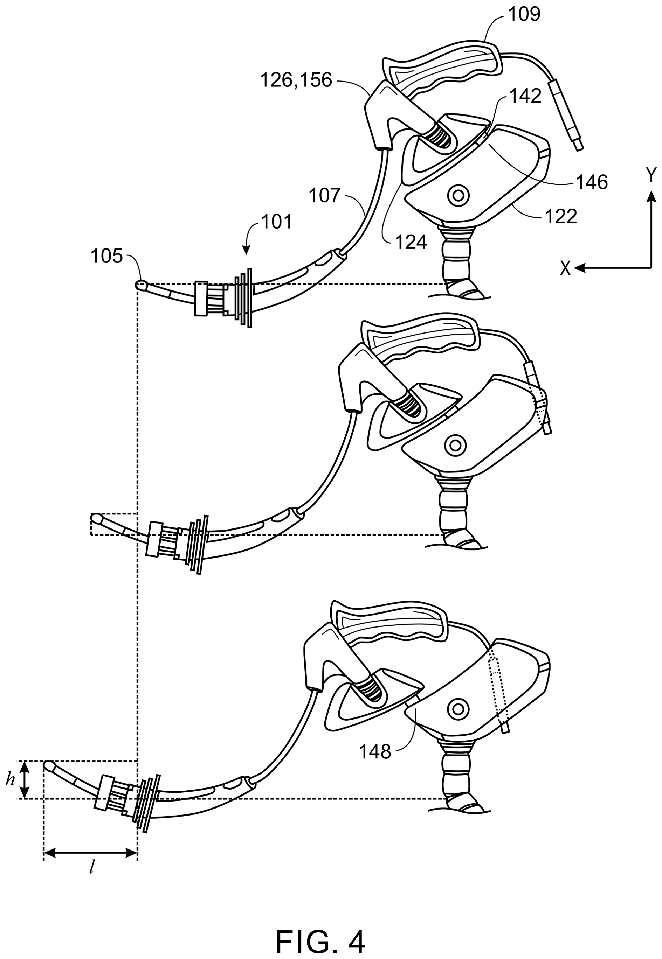

Referring still to FIGS. 2 and 3, the connector 142 is an elongate component (e.g., a rod, a peg, or an extension piece) that extends from a lower surface of the housing 138. A lower end of the connector 142 is shaped such that the lower end is retained within the elongate receptacle 136 of the base 122. The elongate receptacle 136 is formed substantially parallel to a curved profile of the housing 128 in the *xy* plane. The curved shape of the elongate receptacle 136 allows forward and backward movements of the adjustment unit 124 with respect to the horizontal x axis and upward and downward movements of the adjustment unit 124 with respect to the vertical *y* axis. The forward/backward and upward/downward components of the movement together provide the arcuate motion of the adjustment unit 124. Referring to FIG. 4, in some embodiments, the adjustment unit 124 can move forward (e.g., along the *x* axis) from a rearmost position 146 (e.g., defined at the connector 142) to a forward-most position 148 by a total distance *l* of up to about 6 cm to up to about 26 cm. In some embodiments, the adjustment unit 124 can move vertically (e.g., along the *y* axis) by a total distance *h* of up to about 5 cm to up to about 10 cm.

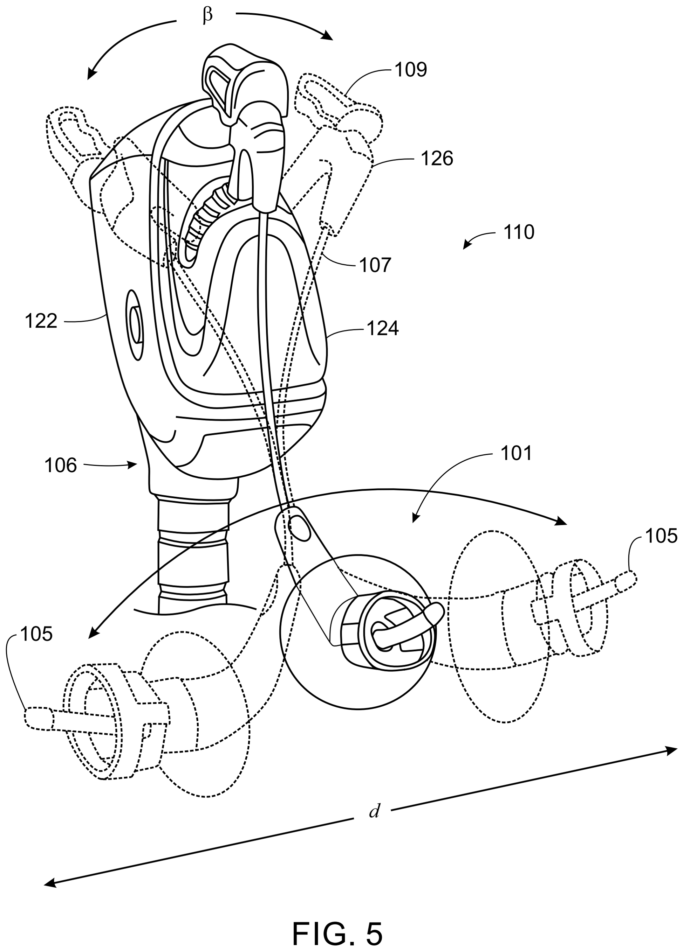

Referring to FIGS. 2 and 5, the rotational element 144 includes a dial 150 and an adapter mount. The adapter mount extends from the dial 150 and is fitted securely within a corresponding receptacle of the adapter 126. The dial 150 is rotatable about an interior pin by an angle *β* of up to about 90 degrees to up to about 270 degrees to adjust an orientation of the adapter 126 and accordingly, the orientation of the uterine manipulator 101 that is secured to the adapter 126. In some embodiments, such an angular span corresponds to a lateral range *d* of the tip 105 of the uterine manipulator 101 of about 6 cm to about 36 cm.

The adapter 126 includes a body 156 that defines the corresponding receptacle and an attachment feature (e.g., a slot, a receptacle, or another attachment feature) to which a shaft 107 of the uterine manipulator 101 can be securely fitted. The adapter 126 also includes a rear connection feature at which the body 156 is attachable to a handle 109 of the uterine manipulator 101. That is, the shaft 107 of the uterine manipulator 101 can be removed from the handle 109 of the uterine manipulator 101 to allow attachment of both components to the adapter 126. In some embodiments, the handle 109 of the uterine manipulator 101 may be discarded in lieu of a handle 109 that may instead be provided as preassembled with the body 156 of the adapter 126.

Once the uterine manipulator 101 has been installed to the adapter 126, the adapter 126 has been secured to the adjustment unit 124, and the positioning arm 104 has been locked in a desired configuration, the remote controller 108 (e.g., a joystick) can be operated at a remote user workstation to robotically manipulate the adjustment unit 124 of the adjustment device 110. For example, the adjustment unit 124 can be moved forward and downward along the elongate receptacle 136 as discussed above, where the downward movement results in an upward movement of the tip 105 of the uterine manipulator 101 due to a curved shape of the shaft 107. Additionally, the dial 150 can be rotated to change an orientation of the adapter 126 and, correspondingly, to change the orientation of the uterine manipulator 101 (e.g., to tilt the uterine manipulator 101 from side to side).

While the adjustment device 110 has been described and illustrated with respect to certain dimensions, sizes, shapes, arrangements, configurations, materials, and methods, in some embodiments, an adjustment device that is otherwise substantially similar in construction and function to the adjustment device 110 may include one or more different dimensions, sizes, shapes, arrangements, configurations, and materials or may be utilized according to different methods.

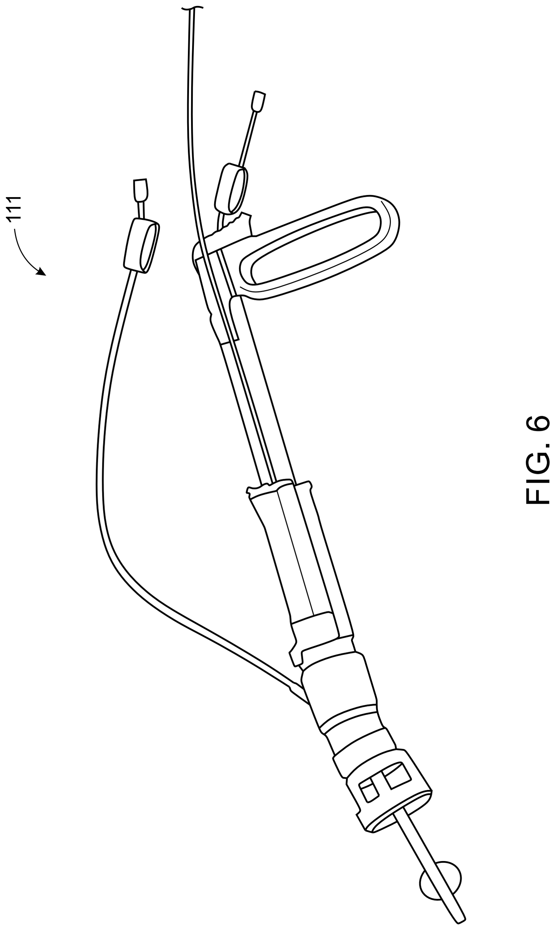

For example, while the adjustment device 110 has been described and illustrated as including the adapter 126 that is designed to mate with the uterine manipulator 101, in some embodiments, an adjustment device that is otherwise substantially similar in construction and function to the adjustment device 110 may alternatively include a different adapter that is designed to mate with a different uterine manipulator, such as the uterine manipulator 111 shown in FIG. 6.

While the adjustment device 110 has been described and illustrated with respect to certain numerical ranges for the parameters *l, h, β,* and *d,* in some embodiments, an adjustment device that is otherwise substantially similar in construction and function to the adjustment device 110 may alternatively be designed to operate according to different numerical ranges for one or more of the parameters *l*, *h, β,* and *d.*

While the adjustment device 110 has been described and illustrated as including the rechargeable battery 132 within the lower base 122, in some embodiments, an adjustment device that is otherwise substantially similar in construction and function to the adjustment device 110 may alternatively include a rechargeable battery within an upper adjustment unit instead of within a lower base.

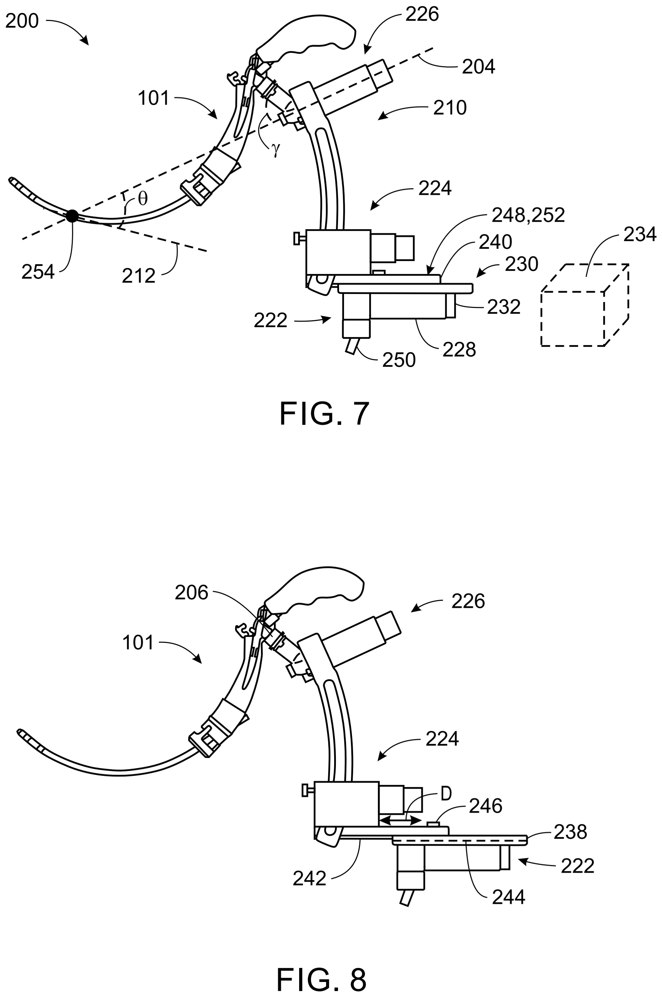

Referring to FIGS. 7 and 8, in some embodiments, an adjustment device 210 may be used alternatively to the adjustment device 110 for locally positioning the uterine manipulator 101 of a positioning system 200. The positioning system 200 is substantially similar in construction and function to the positioning system 100, except that the positioning system 200 includes the adjustment device 210 instead of the adjustment device 110. Accordingly, the positioning system 200 further includes the mounting/powering unit 102, the positioning arm 104, the remote controller 108, and the foot pedal 112 of the positioning system 100, all of which are shown in FIG. 1. The adjustment device 210 includes a device connector 250 by which the adjustment device 210 is fixedly attached to the positioning arm 104.

Once the positioning arm 104 of the positioning system 200 has been locked in a desired configuration to establish a global position of the adjustment device 210, the adjustment device 210 can be operated with the remote controller 108 to fine-tune a position of the uterine manipulator 101, which is supported on the adjustment device 210. The adjustment device 210 also includes a lower motor assembly 222 (e.g., a translation assembly) that is fixedly attached to the upper end 106 of the positioning arm 104, an intermediate motor assembly 224 that is fixedly attached to the lower motor assembly 222, and an upper motor assembly 226 (e.g., an adjustment unit) that is fixedly attached to the intermediate motor assembly 224.

The lower motor assembly 222 is operable to effect linear motion of the uterine manipulator 101 along the x axis (e.g., in both positive and negative horizontal x-axis directions). The motor assembly 222 includes a housing 228, a motor 232 located within the housing 228, and a translation mechanism 230 located at an upper side of the housing 228. The motor 232 drives linear motion of the translation mechanism 230. In some embodiments, the motor 232 is a brushless DC motor with an integrated planetary gear transmission. In some embodiments, one or more components of the adjustment device 210 are coupled to a separate control box 234 that supplies power to the motor 232 (e.g., via a signal cable), that communicates control signals wirelessly to the motor 232, and that effects wired or wireless communication with the remote controller 108 (shown in FIG. 1). In some embodiments, the adjustment device 210 alternatively includes an onboard power supply (e.g., a battery) and an onboard controller that power and control the motor 232 and that communicate with the remote controller 108.

The translation mechanism 230 includes a linear track 238 (e.g., an elongate, substantially straight track) and a linear coupling member 240 that is slidable and translatable within the linear track 238. For example, the linear coupling member 240 includes a lower rail 242 that is formed complementary to an inner profile 244 (e.g., represented by a dashed line 244) of the linear track 238 such that the rail 242 is slidable and translatable linearly along the inner profile 244. The rail 242 is connected or otherwise coupled to the motor 232 such that the linear motion of the rail 242 along the x direction is driven by action of the motor 232.

In some embodiments, the motor assembly 222 optionally includes a position stop 246 that limits an extent to which the linear coupling member 240 can translate in the *+x* direction. In some embodiments, the rail 242 is movable along the *+x* direction by a distance D of up to about 10 cm with respect to the linear track 238. The linear coupling member 240 includes an upper platform 248 from which the lower rail 242 extends and through which the position stop 246 extends. The intermediate motor assembly 224 is mounted to the platform 248 (e.g., along a substantially flat surface 252) such the that intermediate motor assembly 224 is moved linearly due to operation of the lower motor assembly 222. In this way, the lower motor assembly 222 provides the adjustment device 210 with a linear degree of freedom along the x axis.

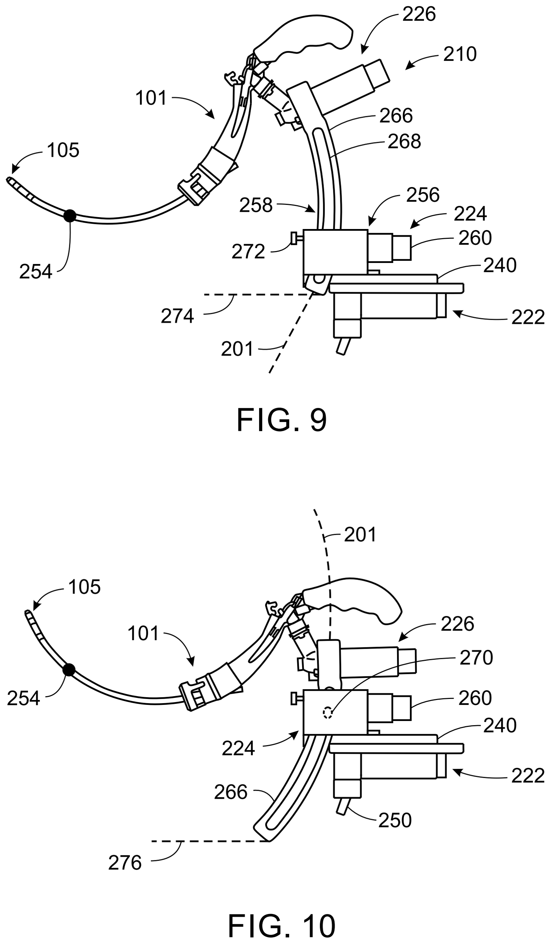

Referring to FIGS. 9 and 10, the intermediate motor assembly 224 is operable to effect arcuate motion (e.g., curvilinear motion with components along both the x and *y* axe) of the uterine manipulator 101 around a reference point 254. The reference point 254 is fixed with respect to the linear coupling member 240 and corresponds to a location of the cervix of the patient's uterus (e.g., a uterus of average depth of about 8 cm). The motor assembly 224 includes a housing 256 that is secured to the platform 248 of the translation mechanism 230, a motor 260 located within the housing 256, and an arcuate movement mechanism 258 that is coupled to the housing 256. The motor 260 drives arcuate motion of the mechanism 258. In some embodiments, the motor 260 is a brushless DC motor with an integrated planetary gear transmission. In some embodiments, the control box 234 (shown in FIG. 7) supplies power to the motor 260 (e.g., via a signal cable), communicates control signals wirelessly to the motor 260, and effects wired or wireless communication with the remote controller 108 (shown in FIG. 1). In alternative embodiments, an onboard power supply (e.g., a battery) and an onboard controller of the adjustment device 210 are provided to power and control the motor 260 and communicate with the remote controller 108.

The arcuate movement mechanism 258 includes an arcuate coupling member 266 (e.g., a support component) that defines an arcuate track 268 (e.g., a curvilinear recess, channel, or slot). The arcuate movement mechanism 258 also includes a gear 270 that is positioned within the housing 256 along the arcuate track 268 and that is coupled to and driven by the motor 260. The gear 270 is coupled to the arcuate track 268 such that operation (e.g., rotation) of the gear 270 against the arcuate track 268 causes the arcuate coupling member 266 to move upward or downward through the housing 256 along an arcuate path 201 that is constrained and defined by the arcuate track 268. The motor assembly 224 further includes a coupling member 272 that secures the arcuate coupling member 266 to the housing 256.

Movement of the arcuate coupling member 266 with respect to (e.g., through) the housing 256 along the path 201 causes the uterine manipulator 101 to undergo a corresponding arcuate motion that maintains the shaft 107 of the uterine manipulator 101 about the reference point 254 at all times. Constraining the motion of the shaft 107 in this manner ensures that (e.g., for a given x-axis position of the housing 256), the tip 105 of the uterine manipulator 101 is positioned appropriately (e.g., at a desired vertical position) within the patient's uterus. In some embodiments, movement of the arcuate coupling member 266 from a first vertical position 274 shown in FIG. 9 to a second vertical position 276 shown in FIG. 10 results in a vertical movement of the tip 105 through a distance of about 3 cm. A desired position (e.g., an operable position) of the tip 105 may be selected by causing the arcuate coupling member 266 to move to any desired vertical position between the first and second vertical positions 274, 276 as the arcuate coupling member 266 moves through the housing 256 along the path 201.

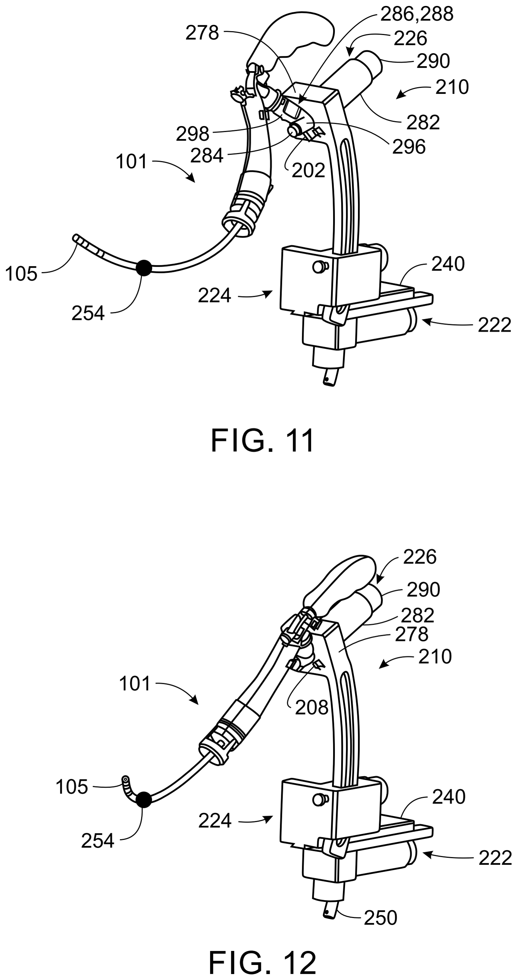

The upper motor assembly 226 is mounted to a support base 278 (shown in FIGS. 11 and 12) of the arcuate coupling member 266 (e.g., at an opening) such that the upper motor assembly 226 is moved arcuately due to operation of the intermediate motor assembly 224. In this way, the intermediate motor assembly 224 provides the adjustment device 210 with degrees of freedom along both the *x* and *y* axes by effecting arcuate motion.

Referring to FIGS. 11 and 12, the upper motor assembly 226 is operable to effect rotational motion of the uterine manipulator 101 about the reference point 254. The motor assembly 226 includes a housing 282 (e.g., a base component) that is mounted to the support base 278 of the arcuate coupling member 266. For example, a connecting rod 284 of the motor assembly 226 extends from the housing 282 through the opening in the support base 278 to a device coupling member 286 of the motor assembly 226 that is coupled to the uterine manipulator 101. The device coupling member 286 and the connecting rod 284 together provide a rotational mechanism 288. The motor assembly 226 further includes a motor 290 located within the housing 282. The motor 290 drives rotational motion of the mechanism 288. In some embodiments, the motor 290 is a brushless DC motor with an integrated planetary gear transmission. In some embodiments, the control box 234 (shown in FIG. 7) supplies power to the motor 290 (e.g., via a signal cable), communicates control signals wirelessly to the motor 290, and effects wired or wireless communication with the remote controller 108 (shown in FIG. 1). In alternative embodiments, an onboard power supply (e.g., a battery) and an onboard controller of the adjustment device 210 are provided to power and control the motor 290 and communicate with the remote controller 108.

The connecting rod 284 of the rotational mechanism 288 is rotatably driven by the motor 290. The device coupling member 286 is fixedly attached to and surrounds the connecting rod 284 such that rotation of the connecting rod 284 causes rotation of the device coupling member 286. The device coupling member 286 includes a substantially cylindrical portion 296 that surrounds the connecting rod 284 and a support arm 298 (e.g., a rotational element) that extends upward from the cylindrical portion 296. In some embodiments, the device coupling member 286 optionally includes a stopping element 202 that extends downward from the cylindrical portion 296. An adaptor 206 that is attached to the uterine manipulator 101 is mounted to the support arm 298. The support arm 298 is also oriented at an acute angle γ (refer to FIG. 7) with respect to a central axis 204 of the motor 290, which in part causes the shaft 107 of the uterine manipulator 101 to intersect the reference point 254.

Owing to the attachment of the cylindrical portion 296 to the connecting rod 284, rotation of the connecting rod 284 causes the support arm 298 to rotate correspondingly from side to side with respect to the reference point 254. In some embodiments, the support base 278 of the arcuate coupling member 266 optionally includes two positional stoppers 208 (only one is visible in FIGS. 11 and 12) that are located oppositely with respect to the central axis 204. The stopping element 202 abuts a positional stopper 208 when the device coupling member 286 reaches a certain degree of rotation such that the stopping elements 202 limit a rotational extent of the device coupling member 286. In some embodiments, the device coupling member 286 is permitted to rotate by up to about 30 degrees to each side of the housing 282 with respect to the central axis 204 of the housing 282.

Referring to FIGS. 7, 11, and 12, the central axis 204 of the motor 290 intersects the reference point 254 (e.g., corresponding to the cervix), thus ensuring that rotation of the uterine manipulator 101 occurs with respect to the cervix. An acute angle θ between the central axis 204 and a line 212 tangent to the shaft 107 of the uterine manipulator 101 and intersecting the reference point 254 is maximized to in order to maximize the rotational extent of the distal tip 105 of the uterine manipulator 101. In some embodiments, the angle θ is about 30 degrees to about 50 degrees. The angle θ can remain fixed because the rotational motor assembly 226 is connected directly to the uterine manipulator 101 (e.g., via the adapter 206). Mounting the motor assembly 226 in a different configuration (e.g., between the lower motor assembly 222 and the intermediate assembly 224) would undesirably cause the angle between the tangent line 212 and the central axis 204 to change as the arcuate motion (discussed with respect to FIGS. 9 and 10) of the uterine manipulator 101 changes, which is unacceptable during a surgical procedure.

The upper motor assembly 224 provides the adjustment device 210 with a rotational degree of freedom about the reference point 254. In some embodiments, the control box 234 (shown in FIG. 7) supplies power to the motor 290, communicates control signals wirelessly to the motor 290, and effects wired or wireless communication with the remote controller 108 (shown in FIG. 1). In alternative embodiments, an onboard power supply (e.g., a battery) and an onboard controller of the adjustment device 210 are provided to power and control the motor 290 and communicate with the remote controller 108.

In some embodiments, the adjustment device 210 may be powered on and off according to operation of the control box 234, and the adjustment device 210 may still optionally include a power selector (e.g., a button or a switch) located on the housing 228 or another component of the adjustment device 210. In alternative embodiments for which the adjustment device 210 includes an onboard power supply (e.g., a battery) and an onboard controller, the adjustment device 210 includes a power selector located on the housing 228 or another component of the adjustment device 210. In some embodiments, the adjustment device 210 weighs about 2 kg to about 4 kg. Example materials from which the housings 228, 256, 282 and components of the mechanisms 230, 258, 288 may be made include steel and aluminum.

While the adjustment device 210 has been described and illustrated with respect to certain dimensions, sizes, shapes, arrangements, configurations, materials, and methods, in some embodiments, an adjustment device that is otherwise substantially similar in construction and function to the adjustment device 210 may include one or more different dimensions, sizes, shapes, arrangements, configurations, and materials or may be utilized according to different methods.

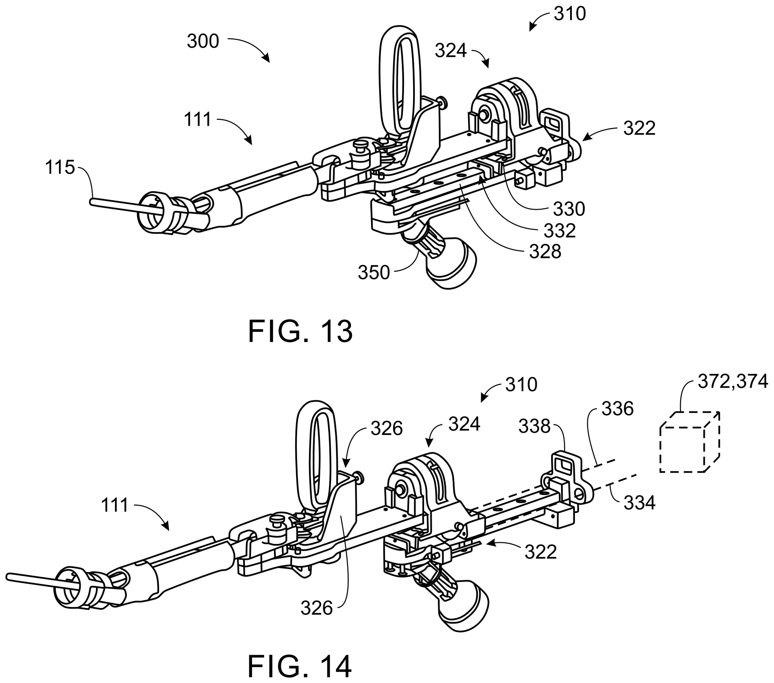

Referring to FIGS. 13 and 14, in some embodiments, an adjustment device 310 may be used for locally positioning the uterine manipulator 111 of a positioning system 300. The positioning system 300 is substantially similar in construction and function to the positioning system 100, except that the positioning system 300 includes the adjustment device 310 instead of the adjustment device 110 and the uterine manipulator 111 instead of the uterine manipulator 101. Accordingly, the positioning system 300 further includes the mounting/powering unit 102, the positioning arm 104, the remote controller 108, and the foot pedal 112 of the positioning system 100, all of which are shown in FIG. 1.

Once the positioning arm 104 of the positioning system 300 has been locked in a desired configuration to establish a global position of the adjustment device 310, the adjustment device 310 can be operated with the remote controller 108 to fine-tune a position of the uterine manipulator 111, which is supported on the adjustment device 310. Accordingly, the adjustment device 310 includes a device connector 350 by which the adjustment device 310 is fixedly attached to the positioning arm 104. The adjustment device 310 also includes a translational assembly 322 that is fixedly attached to the upper end 106 of the positioning arm 104, a rotational assembly 324 that is fixedly attached to the translational assembly 322, and a rotatable receptacle 326 (e.g., a rotatable adjustment unit) that is fixedly attached to the rotational assembly 324.

The translational assembly 322 is operable to effect linear motion of the uterine manipulator 111 along the x axis (e.g., in both positive and negative horizontal x-axis directions). The translational assembly 322 includes a rail 328 that is fixed to the device connector 350, a rear wall 338 that is fixed to an end of the rail 328, and a horizontal support structure 330 (e.g., a carriage) that is slidable linearly along the rail 328. Accordingly, the rail 328 has a substantially rectangular shape, and the horizontal support structure 330 defines a slot 332 with a complementary shape to allow linear movement of the horizontal support structure 330 along the rail 328.

The translational assembly 322 further includes two flexible mechanical actuation cables (e.g., Bowden cables) by which the horizontal support structure 330 can be moved (e.g., pushed forward) in the +*x* direction by one cable 334 (represented by the dashed line 334) and moved (e.g., pulled backward) in the -*x* direction by the other cable 336 (represented by the dashed line 336). Each cable 334, 336 is connected to the horizontal support structure 330 at one end and passes through the rear wall 338 to connect to a remote motor 372 at an opposite end. The remote motor 372 is controlled by associated electronics 374 upon operation of the remote controller 108 to activate the cables 334, 336 for moving the horizontal support structure 330 linearly. In some embodiments, the remote motor 372 is a linear actuator. In some embodiments, the horizontal support structure 330 is movable along the +x direction by a distance of up to about 10 cm with respect to the rail 328. In this way, the translational assembly 322 provides the adjustment device 310 with a linear degree of freedom along the x axis.

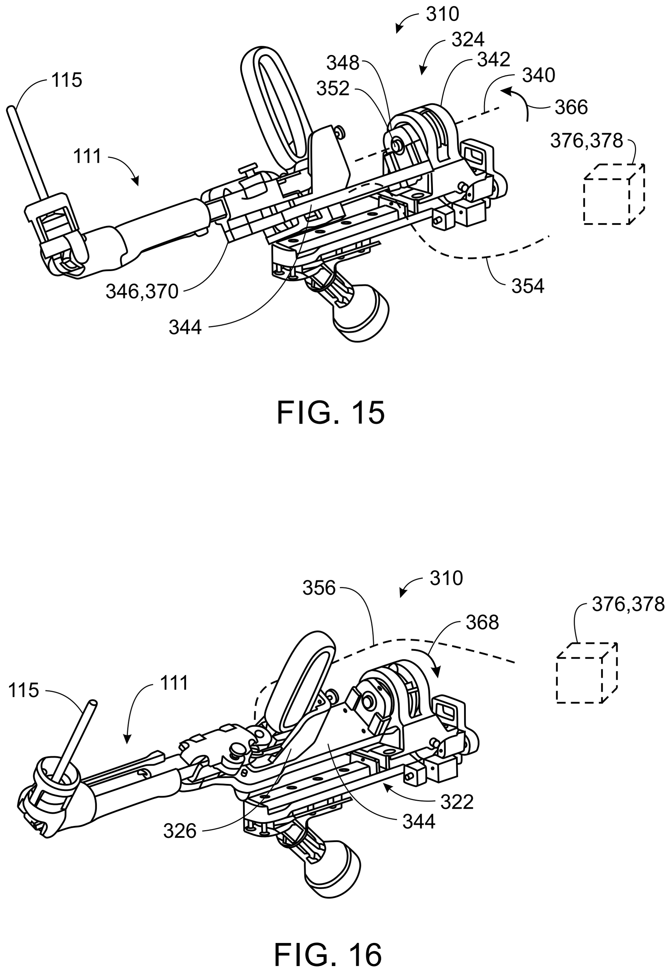

Referring to FIGS. 15 and 16, the rotational assembly 324 that is operable to effect rotational motion of the uterine manipulator 111 about a rotational axis 340 that extends parallel to the x axis and through the patient's cervix. The rotational assembly 324 includes a vertical support structure 342 and a platform 344 (e.g., a rotatable support component) that is coupled to the vertical support structure 342. The platform 344 includes a base 346 that is secured to the uterine manipulator 111 at a distal portion 370 and a coupling wall 348. The rotational assembly 324 includes a pin 352 that couples the coupling wall 348 of the platform 344 to the vertical support structure 342 of the translational assembly 322 to allow the platform 344 to rotate (e.g., pivot) with respect to the translational assembly 322.

The rotational assembly 324 further includes two flexible mechanical actuation cables 354, 356 (e.g., Bowden cables) by which the platform 344 can be rotated from side to side with respect to the rotational axis 340. The flexible cable 354 (represented by the dashed line 354 in FIG. 14) is connected to one side of the platform 344 and can be pulled to move the platform 344 in a first rotational direction 358. The flexible cable 356 (represented by the dashed line 356 in FIG. 13) is connected to the opposite side of the platform 344 and can be pulled to move the platform 344 in a second, opposite rotational direction 360.

Each cable 354, 356 is connected to the platform 346 at one end and is connected to a remote motor 376 at an opposite end. The remote motor 376 is controlled by associated electronics 378 upon operation of the remote controller 108 to activate the cables 354, 356 for rotating the platform 346 about the pin 352. In some embodiments, the remote motor 376 is a linear actuator. In some embodiments, the platform 344 is rotatable to each side of the rotational axis 340 by an angle of up to about 90 degrees. The rotatable receptacle 326 is rotatably coupled to the platform 344. In this way, the rotational assembly 324 provides the adjustment device 310 with a rotational degree of freedom with respect to the rotational axis 340, which extends parallel to the *x* axis.

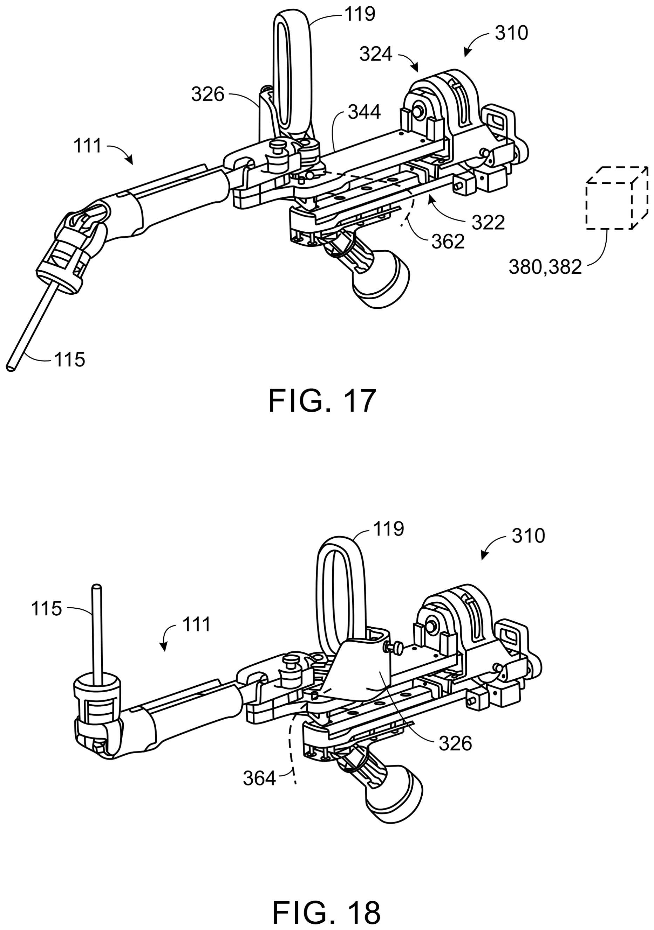

Referring to FIGS. 17 and 18, the rotatable receptacle 326 is operable to effect rotational motion of a distal tip 115 of the uterine manipulator 111 about a transverse axis 123 that extends perpendicular (e.g., along a z axis) to a central axis 117 of the uterine manipulator 111. The distal tip 115 can be articulated (e.g., pivoted) upward by about 90 degrees (shown in FIG. 18) about the transverse axis 123 and downward by about 50 degrees (shown in FIG. 17) about the transverse axis 123 by rotating a handle 119 (e.g., an articulation control element) of the uterine manipulator 111 side to side with respect to the central axis 117 at a pin mechanism 121.

The rotatable receptacle 326 provides a seat (e.g., a holding or support component) in which the handle 119 is securely mounted for rotating the handle 119. The receptacle 326 is equipped with two flexible mechanical actuation cables 362, 364 (e.g., Bowden cables) by which the receptacle 326, carrying the handle 119, can be rotated from side to side with respect to the central axis 117. The flexible cable 362 (represented by the dashed line 362 in FIG. 17) is connected to one side of the receptacle 326 and can be pulled to rotate the receptacle in a first rotational direction 366. The flexible cable 364 (represented by the dashed line 364 in FIG. 18) is connected to the opposite side of the receptacle 326 and can be pulled to rotate the receptacle 326 in a second, opposite rotational direction 368.

Each flexible cable 362, 364 is connected to the receptacle 326 at one end and is connected to a remote motor 380 at an opposite end. The remote motor 380 is controlled by associated electronics 382 upon actuation of the remote controller 108 to activate the cables 362, 364 for rotating the receptacle 326 about the pin mechanism 121. In some embodiments, the remote motor 380 is a linear actuator. In this way, the rotatable receptacle 326 provides the adjustment device 310 with a rotational degree of freedom about the transverse axis 123, which extends perpendicular to the central axis 117 and to the x axis.

In contrast to the uterine manipulator 101, the uterine manipulator 111 includes different distal tips 115 that can be selected for use with uteruses of differing depths. The result is that the cervix remains in the same position relative to the handle 119 such that the adjustment device 310 can adjust the distal tip 115 via the rotational assembly 324 and the rotatable receptacle 326 at the cervix irrespective of the depth of the uterus.

Location of the motors 372, 376, 380 of the adjustment device 310 at remote positions advantageously reduces a weight of the adjustment device 310. In some embodiments, the adjustment device 310 weighs about 0.5 kg to about 3 kg. Example materials from which the translational assembly 322, the rotational assembly 324, and the rotatable receptacle 326 may be made include aluminum and steel.

While the adjustment device 310 has been described and illustrated with respect to certain dimensions, sizes, shapes, arrangements, configurations, materials, and methods, in some embodiments, an adjustment device that is otherwise substantially similar in construction and function to the adjustment device 310 may include one or more different dimensions, sizes, shapes, arrangements, configurations, and materials or may be utilized according to different methods.

For example, while the adjustment device 310 has been described and illustrated as including the rotatable receptacle 326 that is designed to seat the handle 119 of the uterine manipulator 111, in some embodiments, an adjustment device that is otherwise substantially similar in construction and function to the adjustment device 310 may alternatively include a rotatable receptacle 326 that is designed to seat a different rotatable component (e.g., a fitting) of the uterine manipulator 111 that replaces the handle 119.

While the adjustment device 310 has been described and illustrated as including remote motors 372, 376, 380 that provide actuation via the mechanical actuation cables 334, 336, 354, 356, 362, 364, in some embodiments, an adjustment device that is otherwise substantially similar in construction and function to the adjustment device 310 may alternatively include remote motors that provide actuation via hydraulic mechanisms.

While the adjustment device 310 has been described and illustrated as including remote motors 372, 376, 380 that provide actuation via the mechanical actuation cables 334, 336, 354, 356, 362, 364, in some embodiments, an adjustment device that is otherwise substantially similar in construction and function to the adjustment device 310 may alternatively include one or more local motors that are connected directly to local components of the assemblies 322, 324 and to the receptacle 326.

The adjustment device 310 has been described and illustrated as including three sets of two mechanical actuation cables 334, 336; 354, 356; 362, 364, where each cable of each set effects an oppositely directed movement of the mechanism 322, 324, 326, respectively. However, in some embodiments, an adjustment device that is otherwise substantially similar in construction and function to the adjustment device 310 may alternatively include a single flexible mechanical actuation cable for each movement mechanism 322, 324, 326 and that can selectively effect either of two oppositely directed movements. In some embodiments, either cable of a set of two mechanical actuation cables 334, 336; 354, 356; or 362, 364 may be configured to effect either of two alternative oppositely directed movements.

Remotely controlling robotic movements of the adjustment devices 110, 210, 310 can allow the surgeon to position the patient's uterus (e.g., seated on a uterine manipulator 101, 111, which is in turn secured to an adjustment device 110, 210, 310) as desired for optimal cutting and removal access to the uterus without leaving his or her workstation at which the remote controller 108 is operated. Thus, the surgeon can continue visualizing the patient's pelvic region on a display screen at the robotic surgery workstation while the adjustment device 110, 210, 310 is manipulated remotely without disruption. Such remote control of the adjustment devices 110, 210, 310 therefore advantageously results in a streamlined procedure with an improved effectiveness, an improved precision, and a reduced procedural time period.

In some embodiments, any of the adjustment devices 110, 210, 310 may be provided as a modular device that can be retrofitted to or otherwise assembled with a positioning arm 104 of a positioning system 100, 200, 300 at a point of care. In other embodiments, the adjustment device 110, 210, 310 may be preassembled with a positioning arm 104 of a positioning system 100, 200, 300.

While the adjustment devices 110, 210, 310 have been described and illustrated as permitting certain degrees of freedom, in some embodiments, an adjustment device that is otherwise substantially similar in construction and function to any of the adjustment devices 110, 210, 310 may be designed to allow additional or alternative movements and/or degrees of freedom.

Accordingly, other embodiments are also within the scope of the following claims.

### EMBODIMENTS

Although the present invention is defined in the attached claims, it should be understood that the present invention can also (alternatively) be defined in accordance with the following embodiments:
1. An adjustment device for positioning a uterine manipulator, the adjustment device comprising:
   a support component that is movable along an arcuate path defined by the adjustment device; and
   an adjustment unit comprising:
      a base component that is secured to the support component, and
      a rotational element that is secured to the base component and to the uterine manipulator such that the rotational element and the uterine manipulator secured thereto are movable along the arcuate path and rotatable with respect to the support component.
2. The adjustment device of embodiment 1, wherein the adjustment device is configured to be fixedly attached to a positioning arm of a uterine positioning system.
3. The adjustment device of embodiment 2, wherein the adjustment unit is configured to move forward and backward along the arcuate path with respect to the positioning arm.
4. The adjustment device of embodiment 1, further comprising internal electronics that are configured to effect wireless or wired communication with a remote controller.
5. The adjustment device of embodiment 1, further comprising a remote control unit that is configured to power and control one or more movement mechanisms of the adjustment device.
6. The adjustment device of embodiment 1, further comprising a support unit that provides an elongate receptacle, the elongate receptacle receiving the support component and defining the arcuate path.
7. The adjustment device of embodiment 6, wherein the support component comprises a connector having an end that is retained within the elongate receptacle as the adjustment unit moves arcuately along the elongate receptacle.
8. The adjustment device of embodiment 1, wherein the adjustment unit is movable forward and backward along the arcuate path by a total distance of up to about 6 cm to up to about 26 cm.
9. The adjustment device of embodiment 1, wherein the rotational element comprises a dial that is rotatable about a pin, and wherein an angular span of the dial corresponds to a lateral range of a tip of the uterine manipulator of about 6 cm to about 36 cm.
10. The adjustment device of embodiment 1, wherein the support component defines the arcuate path.
11. The adjustment device of embodiment 1, further comprising one or more motors that control arcuate movement of the support component and rotation of the rotational element.
12. The adjustment device of embodiment 1, further comprising a translation assembly that is coupled to and causes linear movement of the support component.
13. The adjustment device of embodiment 1, wherein the adjustment device is configured to maintain rotation of a shaft of the uterine manipulator around a reference point as the support component moves along the arcuate path.
14. The adjustment device of embodiment 13, wherein the reference point is located at a patient's cervix.
15. The adjustment device of embodiment 1, wherein the adjustment device is configured such that rotation of the uterine manipulator by the rotational element is maintained about a reference point.
16. The adjustment device of embodiment 15, wherein the reference point is located at a patient's cervix, and wherein an axis of the adjustment unit intersects the reference point.
17. A positioning system comprising:
   a positioning arm; and
   an adjustment device supported on the positioning arm for positioning a uterine manipulator, the adjustment device comprising:
      a support component that is movable along an arcuate path defined by the adjustment device, and
      an adjustment unit comprising:
         a base component that is secured to the support component, and
         a rotational element that is secured to the base component and to the uterine manipulator such that the rotational element and the uterine manipulator secured thereto are movable along the arcuate path and rotatable with respect to the support component.
18. The positioning system of embodiment 17, wherein the positioning arm defines a global position of the adjustment device and wherein the adjustment device defines a local position of the uterine manipulator.
19. The positioning system of embodiment 17, further comprising:
   a remote controller; and
   a control box that communicates with the remote controller.
20. An adjustment device for positioning a uterine manipulator, the adjustment device comprising:
   a rotatable support component that is secured to the uterine manipulator and rotatable about a rotational axis defined by the adjustment device; and
   a rotatable adjustment unit that is secured to an articulation control element of the uterine manipulator and to the rotatable support component such that the adjustment device is configured to rotate the uterine manipulator with respect to the rotational axis and to articulate a portion of the uterine manipulator with respect to the rotatable support component.
21. The adjustment device of embodiment 20, wherein the rotatable support component is coupled to a linear translation assembly.
22. The adjustment device of embodiment 21, wherein the linear translation assembly comprises a rail and a carriage.
23. The adjustment device of embodiment 20, wherein the linear translation assembly is configured to be fixedly attached to a positioning arm of a uterine positioning system.
24. The adjustment device of embodiment 20, further comprising one or more mechanical actuation cables that are configured to selectively cause the rotatable support component to rotate in opposite rotational directions.
25. The adjustment device of embodiment 24, further comprising a remote motor that is coupled to and controls the one or more mechanical actuation cables.
26. The adjustment device of embodiment 20, further comprising one or more mechanical actuation cables that are configured to selectively cause the rotatable adjustment unit to rotate in opposite rotational directions.
27. The adjustment device of embodiment 26, further comprising a remote motor that is coupled to and controls the one or more mechanical actuation cables.
28. The adjustment device of embodiment 20, wherein the articulation control element comprises a handle and wherein the portion comprises a distal tip.
29. The adjustment device of embodiment 20, wherein the rotational axis intersects a patient's cervix.
30. A positioning system comprising:
   a positioning arm; and
   an adjustment device supported on the positioning arm for positioning a uterine manipulator, the adjustment device comprising:
      a rotatable support component that is secured to the uterine manipulator and rotatable about a rotational axis defined by the adjustment device, and
      a rotatable adjustment unit that is secured to an articulation control element of the uterine manipulator and to the rotatable support component such that the adjustment device is configured to rotate the uterine manipulator with respect to the rotational axis and to articulate a portion of the uterine manipulator with respect to the rotatable support component.

## Claims

1. An adjustment device (210) for positioning a uterine manipulator (101), the adjustment device (210) comprising:
a first motor assembly (224) comprising:
a first housing (256),
an arcuate movement mechanism (258) coupled to the first housing (256) and comprising a support component (266) that defines an arcuate track (268) defining an arcuate path (201) and that is movable through the first housing (256) along the arcuate path (201) defined by the adjustment device (210), and
a first motor (260) located within the first housing (256) and configured to drive an arcuate motion of the arcuate movement mechanism (258); and
a second motor assembly (226) attached to the first motor assembly (224) and comprising:
a second housing (282) that is secured to the support component (266) of the first motor assembly (224),
a rotational mechanism (288) comprising a device coupling member (286) of the second motor assembly (226), the device coupling member (286) including a rotational element (298), wherein the rotational element (298) is secured to the second housing (282) and is secured to the uterine manipulator (101) such that the rotational element (298) and the uterine manipulator (101) secured thereto are movable along the arcuate path (201) and rotatable with respect to the support component (266), and
a second motor (290) located within the second housing (282) and configured to drive a rotational motion of the rotational mechanism (288).

2. The adjustment device (210) of claim 1, wherein the adjustment device (210) is configured to be fixedly attached to a positioning arm (104) of a uterine positioning system (100).

3. The adjustment device of claim 1, further comprising a remote control unit (234) that is configured to power and control one or more movement mechanisms (230, 258, 288) of the adjustment device (210).

4. The adjustment device (210) of claim 1, further comprising a translation assembly (222) that is coupled to and causes linear movement of the support component (266).

5. The adjustment device (210) of claim 1, wherein the adjustment device (210) is configured to maintain rotation of a shaft (107) of the uterine manipulator (101) around a reference point (254) as the support component (266) moves along the arcuate path (201), and wherein the reference point is located at a patient's cervix.

6. The adjustment device (210) of claim 1, wherein the adjustment device (210) is configured such that rotation of the uterine manipulator (101) by the rotational element (298) is maintained about a reference point (254).

7. The adjustment device (210) of claim 6, wherein the reference point (254) is located at a patient's cervix, and wherein an axis (204) of the second motor (290) intersects the reference point (254).

8. The adjustment device (210) of claim 1, wherein the second motor assembly (226) is mounted to a support base (278) of the support component (266) at an opening in the support base (278).

9. The adjustment device (210) of claim 8, wherein the device coupling member (286) includes a stopping element (202), and wherein the support base (278) includes two positional stoppers (208) that are located oppositely with respect to an axis (204) of the second motor (290) such that the stopping element (202) is configured to abut a positional stopper (208) when the device coupling member (286) reaches a certain degree of rotation to limit a rotational extent of the device coupling member (286).

10. The adjust device (210) of claim 8, wherein the rotational mechanism (288) further comprises a connecting rod (284) extending from the second housing (282) through the opening in the support base (278) to the device coupling member (286).

11. The adjustment device (210) of claim 10, wherein the connecting rod (284) is rotationally drivable by the second motor (290), and wherein the device coupling member (286) is fixedly attached to and surrounds the connecting rod (284) such that rotation of the connecting rod (284) causes rotation of the device coupling member (286).

12. The adjustment device (210) of claim 1, wherein the rotational element (298) of the device coupling member (286) comprises a support arm (298) of the device coupling member (286).

13. The adjustment device (210) of claim 12, wherein the support arm (298) is oriented at an acute angle (γ) with respect to an axis (204) of the second motor (290) to cause a shaft (107) of the uterine manipulator (101) to intersect a reference point (254) located at a patient's cervix.

14. The adjustment device (210) of claim 1, wherein the first motor assembly (224) comprises an intermediate motor assembly (224), wherein the second motor assembly (226) comprises an upper motor assembly (226), wherein the adjustment device (210) further comprises a lower motor assembly (222) that is operable to effect a horizontal linear motion of the uterine manipulator (101), wherein the lower motor assembly (222) is fixedly attached to a positioning arm (104) of a uterine positioning system (100), wherein the intermediate motor assembly (224) is fixedly attached to the lower motor assembly (222), and wherein the upper motor assembly (226) is fixedly attached to the intermediate motor assembly (224).

15. A positioning system (200) comprising:
a positioning arm (104); and
the adjustment device (210) of claim 1, wherein the adjustment device (210) is supported on the positioning arm (104) for positioning the uterine manipulator (101).
